# EUROPÄISCHE PATENTANMELDUNG

(11) **EP 0 691 126 A1**
(43) Veröffentlichungstag der Anmeldung: **10.01.1996**
(21) Anmeldenummer: 95109369.9
(22) Anmeldetag: 16.06.1995
(51) Int. Cl.: A61K 7/42, A61K 7/48

(54) **Kosmetische oder dermatologische Zubereitungen, enthaltend alpha-Hydroxy-carbonsäuren und/oder alpha-Ketocarbon-säuren und anorganische Pigmente**

(30) Priorität: 05.07.1994 DE 4423450
(71) Anmelder: Beiersdorf Aktiengesellschaft, D-20245 Hamburg (DE)
(72) Erfinder: Gers-Barlag, Heinrich, Dr., D-25493 Kummerfeld (DE); Hintze, Ulrich, Dr., D-22459 Hamburg (DE); Berlage, Renate, D-22529 Hamburg (DE); Kaden, Waltraud, D-25468 Halstenbek (DE)

(57) **Zusammenfassung**

Kosmetische oder dermatologische Zubereitungen mit einem Gehalt an einer wirksamen Menge an einer oder mehreren α-Hydroxycarbonsäuren der allgemeinen Formel 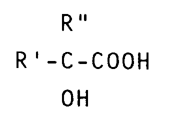
und/oder einer wirksamen Menge an einer oder mehreren α-Ketocarbonsäuren der allgemeinen Formel 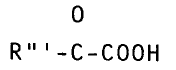
wobei jeweils R', R'' und R''' unabhängig voneinander gewählt werden aus der Gruppe
(b1) H- ,
(b2) verzweigtes oder unverzweigtes C₁₋₂₅-Alkyl-,
(b3) mit einer oder mehreren Carboxylgruppen und/oder Hydroxygruppen und/oder Aldehydgruppen und/oder Oxogruppen (Ketogruppen) substituiertes verzweigtes oder unverzweigtes C₁₋₂₅-Alkyl-
(b4) Phenyl-,
(b5) mit einer oder mehreren Carboxylgruppen und/oder Hydroxygruppen und/oder verzweigten und/oder unverzweigten C₁₋₂₅-Alkylgruppen substituiertes Phenyl-,
oder wobei das α-Kohlenstoffatom der α-Hydroxycarbonsäure mit R' und R'' zusammen eine
(b6) unsubstituierte Cycloalkylgruppe mit 3 bis 7 Ringatomen oder eine
(b7) mit einer oder mehreren Carboxylgruppen und/oder Hydroxygruppen und/oder Oxogruppen (Ketogruppen) und/oder verzweigten und/oder unverzweigten C₁₋₂₅-Alkylgruppen substituierte Cycloalkylgruppe mit 3 bis 7 Ringatomen
ausbildet und
wobei die α-Hydroxycarbonsäure oder die α-Hydroxycarbonsäuren oder die α-Ketocarbonsäure oder die α-Ketocarbonsäuren gegebenenfalls in Form ihrer physiologisch verträglichen Salze und/oder Ethylester und/oder Methylester vorliegen können
und hydrophoben anorganischen Mikropigmenten.

## Beschreibung

Kosmetische oder dermatologische Zubereitungen, enthaltend α-Hydroxycarbonsäuren und/oder α-Ketocarbonsäuren und anorganische Pigmente

Die vorliegende Erfindung betrifft Zubereitungen auf dem Gebiete der kosmetischen und dermatologischen Hautpflege, insbesondere solche Zubereitungen mit niedrigem Reizpotential bzw. kosmetische oder dermatologische Zubereitungen zur Prophylaxe, Linderung oder Beseitigung von Hautreizung.

Insbesondere betrifft die Erfindung Zubereitungen mit extrem niedrigem sogenanntem "Stinging Potential".

Unter Hautpflege ist in erster Linie zu verstehen, daß die natürliche Funktion der Haut als Barriere gegen Umwelteinflüsse (z.B. Schmutz, Chemikalien, Mikroorganismen) und gegen den Verlust von körpereigenen Stoffen (z.B. Wasser, natürliche Fette, Elektrolyte) gestärkt oder wiederhergestellt wird.

Wird diese Funktion gestört, kann es zu verstärkter Resorption toxischer oder allergener Stoffe oder zum Befall von Mikroorganismen und als Folge zu toxischen oder allergischen Hautreaktionen kommen.

Ziel der Hautpflege ist es ferner, den durch tägliche Waschen verursachten Fett- und Wasserverlust der Haut auszugleichen. Dies ist gerade dann wichtig, wenn das natürliche Regenerationsvermögen nicht ausreicht. Außerdem sollen Hautpflegeprodukte vor Umwelteinflüssen, insbesondere vor Sonne und Wind, schützen und die Zeichen der Hautalterung verzögern.

Medizinische topische Zusammensetzungen enthalten in der Regel ein oder mehrere Medikamente in wirksamer Konzentration. Der Einfachheit halber wird zur sauberen Unterscheidung zwischen kosmetischer und medizinischer Anwendung und entsprechenden Produkten auf die gesetzlichen Bestimmungen der Bundesrepublik Deutschland verwiesen (z.B. Kosmetikverordnung, Lebensmittel- und Arzneimittelgesetz).

Die Epidermis ist reich mit Nerven und Nervenendapparaten wie Vater-Pacini-Lamellenkörpern, Merkel-Zell-Neuritenkomplexen und freien Nervenendigungen für Schmerz-, Kälte-, Wärmeempfindung und Juckreiz ausgestattet.

Bei Menschen mit sensibler, empfindlicher oder verletzlicher Haut kann ein mit "Stinging" (〈engl.〉 "to sting" = verletzen, brennen, schmerzen) bezeichnetes neurosensorisches Phänomen beobachtet werden. Diese "sensible Haut" unterscheidet sich grundsätzlich von "trockener Haut" mit verdickten und verhärteten Hornschichten.

Typische Reaktionen des "Stinging" bei sensibler Haut sind Rötung, Spannen und Brennen der Haut sowie Juckreiz.

Als neurosensorisches Phänomen ist der Juckreiz bei atopischer Haut anzusehen, sowie Juckreiz bei Hauterkrankungen.

"Stinging"-Phänomene können als kosmetisch zu behandelnde Störungen angesehen werden. Starker Juckreiz dagegen, insbesondere bei Atopie auftretendes starkes Hautjucken, kann auch als schwerwiegendere dermatologische Störung bezeichnet werden.

Typische, mit den Begriffen "Stinging" oder "empfindlicher Haut" in Verbindung gebrachte, störende neurosensorische Phänomene sind Hautrötung, Kribbeln, Prickeln, Spannen und Brennen der Haut und Juckreiz. Sie können durch stimulierende Umgebungsbedingungen z.B. Massage, Tensideinwirkung, Wettereinfluß wie Sonne, Kälte, Trockenheit, aber auch feuchte Wärme, Wärmestrahlung und UV-Strahlung, z.B. der Sonne, hervorgerufen werden.

In "Journal of the Society of Cosmetic Chemists" 28, S.197 - 209 (Mai 1977) beschreiben P.J.Frosch und A.M.Kligman eine Methode zur Abschätzung des "Stinging-Potentials" topisch verabreichter Substanzen. Als positive Substanzen werden hier z.B. Milchsäure und Brenztraubensäure eingesetzt.

Nach bisherigen Erkenntnissen tritt eine derartige Empfindlichkeit gegenüber ganz bestimmten Substanzen individuell unterschiedlich auf. Dies bedeutet, eine Person, die bei Kontakt mit einer Substanz, beispielweise Milchsäure "Stingingeffekte" erlebt, wird sie mit hoher Wahrscheinlichkeit bei jedem weiteren Kontakt wiederholt erleben. Der Kontakt mit anderen "Stingern" kann aber auch bei ein und derselben Person auch ohne jede Reaktion verlaufen.

Die kosmetische und dermatologische Verwendung von α-Hydroxycarbonsäuren ist an sich bekannt. Beispielsweise wird Citronensäure zur Pufferung kosmetischer und/oder dermatologischer Zubereitungen, aber auch als Synergist für Antioxidantien in der Haut- und Haarkosmetik verwendet.

DE-OS 42 04 321 beschreibt die Verwendung längerkettiger α-Hydroxycarbonsäuren als Wirkstoffe für kosmetische Desodorantien.

Die Einsatzmenge der α-Hydroxycarbonsäuren ist jedoch nicht unbegrenzt, da bei empfindlichen Personen bereits bei Konzentrationen unterhalb von 0,5 Gew.-% das vorab beschriebene "Stinging" auftreten kann.

Da es, wie vorab beschrieben, aber wünschenswert ist, auch empfindlichen Personen die kosmetische oder dermatologische Verabreichung von α-Hydroxycarbonsäuren zu ermöglichen, war es eine Aufgabe der vorliegenden Erfindung, kosmetische oder dermatologische Zubereitungen zu entwickeln, welche zwar α-Hydroxy- bzw. α-Ketocarbonsäuren enthalten, sich aber durch ein extrem niedriges "Stinging-Potential" auszeichnen, günstigenfalls praktisch frei von "Stingingeffekten" sein sollten.

Aufgabe war also, den Nachteilen des Standes der Technik Abhilfe zu schaffen. Insbesondere war Aufgabe der vorliegenden Erfindung, kosmetische oder dermatologische Zubereitungen zur Verfügung zu stellen, in welche einesteils die vorteilhaften Eigenschaften der α-Hydroxy- bzw. α-Ketocarbonsäuren genutzt werden können, ohne daß aber der Nachteil etwaiger Unverträglichkeiten, etwa des Stingings auftreten würde.

Erstaunlicherweise hat sich herausgestellt, und darin liegt die Lösung dieser Aufgaben begründet, daß kosmetische oder dermatologische Zubereitungen mit einem Gehalt an einer wirksamen Menge an einer oder mehreren α-Hydroxycarbonsäuren der allgemeinen Formel 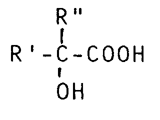
und/oder einer wirksamen Menge an einer oder mehreren α-Ketocarbonsäuren der allgemeinen Formel 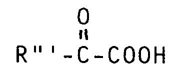
wobei jeweils R', R'' und R''' unabhängig voneinander gewählt werden aus der Gruppe
(b1) H- ,
(b2) verzweigtes oder unverzweigtes C₁₋₂₅-Alkyl-,
(b3) mit einer oder mehreren Carboxylgruppen und/oder Hydroxygruppen und/oder Aldehydgruppen und/oder Oxogruppen (Ketogruppen) substituiertes verzweigtes oder unverzweigtes C₁₋₂₅-Alkyl-
(b4) Phenyl-,
(b5) mit einer oder mehreren Carboxylgruppen und/oder Hydroxygruppen und/oder verzweigten und/oder unverzweigten C₁₋₂₅-Alkylgruppen substituiertes Phenyl-,
oder wobei das α-Kohlenstoffatom der α-Hydroxycarbonsäure mit R' und R'' zusammen eine
(b6) unsubstituierte Cycloalkylgruppe mit 3 bis 7 Ringatomen oder eine
(b7) mit einer oder mehreren Carboxylgruppen und/oder Hydroxygruppen und/oder Oxogruppen (Ketogruppen) und/oder verzweigten und/oder unverzweigten C₁₋₂₅-Alkylgruppen substituierte Cycloalkylgruppe mit 3 bis 7 Ringatomen
ausbildet und
wobei die α-Hydroxycarbonsäure oder die α-Hydroxycarbonsäuren oder die α-Ketocarbonsäure oder die α-Ketocarbonsäuren gegebenenfalls in Form ihrer physiologisch verträglichen Salze und/oder Ethylester und/oder Methylester vorliegen können
und hydrophoben anorganischen Mikropigmenten den Nachteilen des Standes der Technik Abhilfe schaffen.

Es hat sich erstaunlicherweise vorausgestellt, und darin liegt die Lösung der Aufgabe begründet, daß
bzw. die Verwendung von Gemischen aus hydrophoben anorganischen Pigmenten und α-Hydroxycarbonsäuren und/oder α-Ketocarbonsäuren in kosmetischen oder dermatologischen Zubereitungen zur Verhinderung des "Stingings",
bzw. die Verwendung hydrophober anorganischer Pigmente zur Verhinderung des "Stingings" von α-Hydroxycarbonsäuren und/oder α-Ketocarbonsäuren in kosmetischen oder dermatologischen Zubereitungen
den Mißständen des Standes der Technik abhelfen.

Es war nicht vorherzusehen gewesen, daß bei Verwendung der erfindungsgemäßen Gemische nicht nur das "Stinging-Potential" der α-Hydroxycarbonsäuren bzw. α-Ketocarbonsäuren selbst für empfindliche Personen praktisch auf Null reduziert werden, sondern daß darüberhinaus die volle Aktivität der α-Hydroxycarbonsäuren bzw. α-Ketocarbonsäuren erhalten bleiben würde.

Es wird vermutet, daß die erfindungsgemäßen α-Hydroxycarbonsäuren bzw. α-Ketocarbonsäuren an die Oberfläche der hydrophoben anorganischen Pigmente adsorbiert werden.

Zwar beschreiben M.Schmidt und S.G.Steinemann in "XPS studies of amino acids adsorbed on titanium dioxide surfaces", Fresenius' Journal of Analytical Chemistry (1991) 341, S.412 - 415, daß gewisse Aminosäuren leicht von der Oberfläche von Titandioxidpartikeln adsorbiert werden. Ein Hinweis auf die vorliegende Erfindung und ihre vorteilhaften Eigenschaften liefert diese Arbeit jedoch keineswegs. Zudem ist das TiO₂, welches a.a.O. zitiert wird, nicht hydrophob.

Im Gegenteil hätte die vorabzitierte Arbeit vermuten lassen, daß durch eine Adsorption der erfindungsgemäßen α-Hydroxycarbonsäuren bzw. α-Ketocarbonsäuren deren erwünschte Aktivität vermindert wird.

In unerwarteter Weise hat sich jedoch herausgestellt, daß die Aktivität (die sich beispielsweise in den ausgezeichneten Werten für die Hautfeuchtigkeit nach Anwendung Milchsäure enthaltender Formulierungen äußert) kein Unterschied zwischen Formulierungen ergibt, welche erfindungsgemäße Pigmente enthalten und solchen, welche frei davon sind.

Ferner ist überraschend, daß gerade hydrophobierte anorganische Pigmente die erfindungsgemäßen vorteilhaften Eigenschaften aufweisen, da viele der erfindungsgemäßen α-Hydroxycarbonsäuren bzw. α-Ketocarbonsäuren eher als ausgesprochen hydrophile Substanzen zu bezeichnen sind.

Die erfindungsgemäßen α-Hydroxycarbonsäuren werden vorteilhaft gewählt aus folgenden Substanzklassen:
(b2) α-Hydroxyfettsäuren, wobei diese wiederum besonders vorteilhaft aus der Gruppe der C₁₀₋₁₈-Alkylcarbonsäuren gewählt werden,
(b3) α-Hydroxyzuckersäuren, aliphatische α-Hydroxyfruchtsäuren,
(b4) unsubstituierte aromatische α-Hydroxycarbonsäuren (z.B. Mandelsäure) bzw.
(b5) substituierte aromatische α-Hydroxycarbonsäuren.

Die unter Punkt (b2) fallenden α-Hydroxyfettsäuren werden besonders vorteilhaft gewählt aus der Gruppe
- α-Hydroxycarbonsäuren, gemäß der Formel 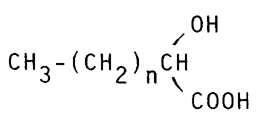 und/oder
- α-Hydroxy-isocarbonsäuren, gemäß der Formel 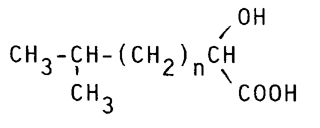 und/oder
- α-Hydroxy-anteisocarbonsäuren, gemäß der Formel 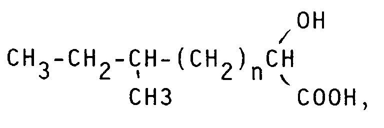
wobei n jeweils eine Zahl von 7 bis 31 darstellt,
Besonders vorteilhaft im Sinne der vorliegenden Erfindung ist, α-Hydroxycarbonsäuren zu verwenden, welche C₁₆-Körper darstellen, die also am α-Kohlenstoffatom eine verzweigte oder unverzweigte C₁₄H₂₉-Kette tragen.

Vorteilhaft ist weiter, Gemische solcher aliphatischen α-Hydroxycarbonsäuren, insbesondere in Form von Wollwachssäuregemischen zu verwenden, in welchen der Gehalt an α-Hydroxycarbonsäuren 20 - 30 Gew.-%, bezogen auf die Gesamtzusammensetzung, beträgt.

Die unter Punkt (b3) fallenden α-Hydroxyzuckersäuren werden besonders vorteilhaft gewählt aus der Gruppe der
- Aldonsäuren, z.B. Gluconsäure, Galactonsäure
- Aldarsäuren, z.B. Glucarsäure, Galactarsäure (aber auch die Fruchtsäure Weinsäure, die ebenfalls unter die Definition der Aldarsäure fällt)
- Uronsäuren, z.B. Glucuronsäure, Galacturonsäure
- Glycerinsäure
Die unter Punkt (b3) fallenden aliphatischen α-Hydroxyfruchtsäuren werden besonders vorteilhaft gewählt aus der Gruppe Äpfelsäure, Milchsäure, Citronensäure, Weinsäure.

Äpfelsäure (Hydroxybernsteinsäure) ist durch folgende chemische Struktur gekennzeichnet: 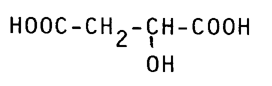
Milchsäure (2-Hydroxypropansäure) ist durch folgende chemische Struktur gekennzeichnet: 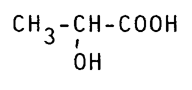
Citronensäure (2-Hydroxy-1,2,3-propantricarbonsäure) ist durch folgende chemische Struktur gekennzeichnet: 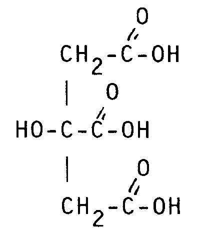
Weinsäure (Dihydroxybernsteinsäure) ist durch folgende chemische Struktur gekennzeichnet: 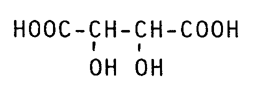
Bevorzugte α-Ketocarbonsäure ist die Brenztraubensäure (α-Oxopropansäure). Sie zeichnet sich durch folgende Struktur aus: 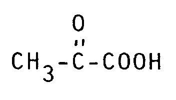
Erfindungsgemäße kosmetische und dermatologische Zubereitungen enthalten bevorzugt anorganische Pigmente auf Basis von Metalloxiden und/oder anderen in Wasser schwerlöslichen oder unlöslichen Metallverbindungen, insbesondere der Oxide des Titans (TiO₂), Zinks (ZnO), Eisens (z.B. Fe₂O₃), Zirkoniums (ZrO₂), Siliciums (SiO₂), Mangans (z.B. MnO), Aluminiums (Al₂O₃), Cers (z.B. Ce₂O₃), Mischoxiden der entsprechenden Metalle sowie Abmischungen aus solchen Oxiden. Besonders bevorzugt handelt es sich um Pigmente auf der Basis von TiO₂.

Voraussetzung für die Verwendbarkeit anorganischer Pigmente für die erfindungsgemäßen Zwecke ist natürlich die kosmetische bzw. dermatologische Unbedenklichkeit der zugrundeliegenden Substanzen.

Im wesentlichen unerheblich für die vorliegende Erfindung ist dabei, in welchen Modifikationen solche Metalloxide vorliegen. TiO₂ beispielsweise kommt in der Natur in drei Hauptmodifikationen (Rutil, Anatas und Brookit) vor, welche grundsätzlich alle gleichermaßen geeignet sind. Ähnliches gilt für die Modifikationen der Eisenoxide usw.

Vorteilhaft ist, den Partikeldurchmesser der verwendeten Pigmente kleiner als 100 nm zu wählen.

Erfindungsgemäß liegen die anorganischen Pigmente in hydrophober Form vor, d.h., daß sie oberflächlich wasserabweisend behandelt sind. Diese Oberflächenbehandlung kann darin bestehen, daß die Pigmente nach an sich bekannten Verfahren mit einer dünnen hydrophoben Schicht versehen werden.

Eines solcher Verfahren besteht beispielsweise darin, daß die hydrophobe Oberflächenschicht nach einer Reaktion gemäß

n TiO₂ + m (RO)₃Si-R' -> n TiO₂ (oberfl.)

erzeugt wird. n und m sind dabei nach Belieben einzusetzende stöchiometrische Parameter, R und R' die gewünschten organischen Reste. Beispielsweise in Analogie zu DE-OS 33 14 742 dargestellte hydrophobisierte Pigmente sind von Vorteil.

Vorteilhafte TiO₂-Pigmente sind beispielsweise unter den Handelsbezeichnungen T 805 (DEGUSSA) oder M 262 (KEMIRA) oder M 160 (KEMIRA) oder MT 100 T (TAYCA) erhältlich.

Vorteilhafte SiO₂-Pigmente können aus der Reihe der unter den Handelsbezeichnungen AEROSIL (DEGUSSA) vertriebenen hydrophoben Pigmente, beispielsweise AEROSIL R 812 oder AEROSIL R 972, gewählt werden.

Erfindungsgemäße Zubereitungen sind vorteilhaft durch einen Gehalt von 0,1 bis 10 Gew.-%, insbesondere 0,5 - 5,0 Gew.-%, an α-Hydroxycarbonsäuren bzw. α-Ketocarbonsäuren und/oder einen Gehalt von 0,1 bis 10 Gew.-%, insbesondere 0,5 - 5,0 Gew.-% an hydrophoben anorganischen Pigmenten gekennzeichnet, jeweils bezogen auf das Gesamtgewicht der Zusammensetzung.

Die Herstellung erfindungsgemäßer Zubereitungen geschieht nach den üblichen, dem Fachmanne geläufigen Regeln. Vorteilhaft liegen die erfindungsgemäßen Zubereitungen in Form von Emulsionen, bevorzugt O/W-Emulsionen vor. Es ist aber auch möglich und erfindungsgemäß gegebenenfalls vorteilhaft, andere Formulierungsarten zu wählen, beispielsweise Hydrodispersionen, Gele, Öle, Multiple Emulsionen, beispielsweise in Form von W/O/W- oder O/W/O-Emulsionen, wasserfreie Salben bzw. Salbengrundlagen usw.

In einfachen Emulsionen liegen in der einen Phase feindisperse, von einer Emulgatorhülle umschlossene Tröpfchen der zweiten Phase (Wassertröpfchen in W/O- oder Lipidvesikel in O/W-Emulsionen) vor. In einer multiplen Emulsion (zweiten Grades) hingegen sind in solchen Tröpfchen feiner disperse Tröpfchen der ersten Phase emulgiert. Auch in diesen Tröpfchen wiederum können noch feiner disperse Tröpfchen vorliegen (multiple Emulsion dritten Grades) und so fort.

So wie man also bei den einfachen Emulsionen von W/O- oder O/W-Emulsionen spricht (Wasser-in-Oel oder Oel-in-Wasser), gibt es bei multiplen Emulsionen W/O/W-, O/W/O-, O/W/O/W-, W/O/W/O-Emulsionen und so fort.

Multiple Emulsionen, bei welchen die jeweiligen inneren und äußeren Wasserphasen oder inneren und äußeren Ölphasen unterschiedlich geartet sind (also z.B. W/O/W'- und O/W/O' -Emulsionen), sind der Präparation durch Zweitopfverfahren zugängig. Solche Emulsionen, in welchen die inneren und äußeren Wasser- bzw. Ölphasen nicht unterschiedlich geartet sind, sind sowohl durch Ein- als auch durch Zweitopfverfahren erhältlich.

Die multiplen Emulsionen zweiten Grades werden gelegentlich als "bimultiple Systeme", solche dritten Grades als "trimultiple Systeme" usw., bezeichnet (W.Seifriz, Studies in Emulsions, J.Phys.Chem., 29 (1925) 738 - 749).

Verfahren zur Herstellung multipler Emulsionen sind dem Fachmann an sich geläufig. So gibt es Zweitopfverfahren, in welchen eine einfache Emulsion (z.B. eine W/O-Emulsion) vorgelegt und durch Zugabe einer weiteren Phase (z.B. eine Wasserphase) mit einem entsprechenden Emulgator (z.B. ein O/W-Emulgator) in eine multiple Emulsion (z.B. eine W/O/W-Emulsion) überführt wird.

Ein zweites Verfahren besteht darin, Emulgatorgemische mit einer Ölphase und einer Wasserphase in einem Eintopfverfahren in eine multiple W/O/W-Emulsion zu überführen. Die Emulgatoren werden in der Ölphase gelöst und mit der Wasserphase vereinigt. Voraussetzung für ein solches Verfahren ist, daß die HLB-Werte (HLB = Hydrophil-Lipophil-Balance) der eingesetzten einzelnen Emulgatoren sich deutlich voneinander unterscheiden.

Hydrodispersionen stellen Dispersionen einer flüssigen, halbfesten oder festen inneren (diskontinuierlichen) Lipidphase in einer äußeren wäßrigen (kontinuierlichen) Phase dar.

Im Gegensatze zu O/W-Emulsionen, die sich durch eine ähnliche Phasenanordnung auszeichnen, sind Hydrodispersionen aber im wesentlichen frei von Emulgatoren. Hydrodispersionen stellen, wie im übrigen auch Emulsionen metastabile Systeme dar, und sind geneigt, in einen Zustand zweier in sich zusammenhängender diskreter Phasen überzugehen. In Emulsionen verhindert die Wahl eines geeigneten Emulgators die Phasentrennung.

Bei Hydrodispersionen einer flüssigen Lipidphase in einer äußeren wäßrigen Phase kann die die Stabilität eines solchen Systems beispielsweise dadurch gewährleistet werden, daß in der wäßrigen Phase ein Gelgerüst aufgebaut wird, in welchem die Lipidtröpfchen stabil suspendiert sind.

Es ist möglich und vorteilhaft, das oder die hydrophoben anorganischen Pigmente und die α-Hydroxycarbonsäuren bzw. α-Ketocarbonsäuren zu jedem beliebigen Zeitpunkte der Emulsionsherstellung dem Emulsionsgemisch zuzugeben. Dabei können Pigment oder Pigmente und α-Hydroxycarbonsäuren bzw. α-Ketocarbonsäuren sowohl getrennt als auch bereits miteinander vereinigt dem Emulsionsgemisch zugegeben werden.

Besonders vorteilhafte Zubereitungen werden ferner erhalten, wenn die erfindungsgemäßen Wirkstoffe mit Antioxidantien kombiniert werden. Erfindungsgemäß enthalten die Zubereitungen vorteilhaft eines oder mehrere Antioxidantien. Als günstige, aber dennoch fakultativ zu verwendende Antioxidantien alle für kosmetische und/oder dermatologische Anwendungen geeigneten oder gebräuchlichen Antioxidantien verwendet werden.

Besonders vorteilhaft werden die Antioxidantien gewählt aus der Gruppe bestehend aus
Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. α-Carotin, β-Carotin, Lycopin) und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, gamma-Linoleyl-, Cholesteryl - und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Pentat-, Hexa-, Heptahioninsulfoximin) in sehr geringen verträglichen Dosierungen (z.B. pmol bis µmol/kg), ferner (Metall)-Chelatoren (z.B. α-Hydroxyfettsäuren, Palmitinsäure, Phytinsäure, Lactoferrin), α-Hydroxysäuren (z.B. Zitronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z.B. gamma-Linolensäure, Linolsäure, Ölsäure), Folsäure und deren Derivate, Ubichinon und Ubichinol deren Derivate, Vitamin C und Derivate (z.B. Ascorbylpalmitate, Mg - Ascorbylphosphate, Ascorbylacetate), Tocopherole und Derivate (z.B. Vitamin E - acetat), Vitamin A und Derivate (Vitamin A - palmitat) sowie Konyferylbenzoat des Benzoeharzes, Rutinsäure und deren Derivate, Ferulasäure und deren Derivate, Butylhydroxytoluol, Butylhydroxyanisol, Furalglucitol, Nordihydroguajakharzsäure, Nordihydroguajaretsäure, Trihydroxybutyrophenon, Harnsäure und deren Derivate, Mannose und deren Derivate, Zink und dessen Derivate (z.B. ZnO, ZnSO₄) Selen und dessen Derivate (z.B. Selenmethionin), Stilbene und deren Derivate (z.B. Stilbenoxid, Trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Besonders vorteilhaft im Sinne der vorliegenden Erfindung können öllösliche Antioxidantien eingesetzt werden.

Die Menge der Antioxidantien (eine oder mehrere Verbindungen) in den Zubereitungen beträgt vorzugsweise 0,001 bis 30 Gew.-%, besonders bevorzugt 0,05 - 20 Gew.-%, insbesondere 1 - 10 Gew.-%, bezogen auf das Gesamtgewicht der Zubereitung.

Sofern Vitamin E und/oder dessen Derivate das oder die Antioxidantien darstellen, ist vorteilhaft, deren jeweilige Konzentrationen aus dem Bereich von 0,001 - 10 Gew.-%, bezogen auf das Gesamtgewicht der Formulierung, zu wählen.

Sofern Vitamin A, bzw. Vitamin-A-Derivate, bzw. Carotine bzw. deren Derivate das oder die Antioxidantien darstellen, ist vorteilhaft, deren jeweilige Konzentrationen aus dem Bereich von 0,001 - 10 Gew.-%, bezogen auf das Gesamtgewicht der Formulierung, zu wählen.

Es war nicht vorauszusehen gewesen, daß die erfindungsgemäße Kombination aus α-Hydroxycarbonsäuren bzw. α-Ketocarbonsäuren und hydrophoben anorganischen Pigmenten mit Antioxidantien neurosensorische Überempfindlichkeit und Juckreiz vermindern. Ferner war nicht vorauszusehen gewesen, daß sie zu hautverträglichen Produkten führen bzw. deren Verträglichkeit steigern und bei gesunder Haut nicht in die hauteigene Mikroflora eingreifen.

Die erfindungsgemäßen kosmetischen Zubereitungen können kosmetische Hilfsstoffe enthalten, wie sie üblicherweise in solchen Zubereitungen verwendet werden, z.B. Konservierungsmittel, Bakterizide, desodorierend wirkende Substanzen, Antitranspirantien, Insektenrepellentien, Vitamine, Mittel zum Verhindern des Schäumens, Farbstoffe, Pigmente mit färbender Wirkung, Verdickungsmittel, weichmachende Substanzen, anfeuchtende und/oder feuchhaltende Substanzen, Fette, Öle, Wachse oder andere übliche Bestandteile einer kosmetischen Formulierung wie Alkohole, Polyole, Polymere, Schaumstabilisatoren, Elektrolyte, organische Lösungsmittel oder Silikonderivate.

Vorteilhaft können die erfindungsgemäßen Zubereitungen außerdem Substanzen enthalten, die UV-Strahlung im UVB-Bereich absorbieren, wobei die Gesamtmenge der Filtersubstanzen z.B. 0,1 Gew.-% bis 30 Gew.-%, vorzugsweise 0,5 bis 10 Gew.-%, insbesondere 1 bis 6 Gew.-% beträgt, bezogen auf das Gesamtgewicht der Zubereitung, um kosmetische Zubereitungen zur Verfügung zu stellen, die die Haut vor dem gesamten Bereich der ultravioletten Strahlung schützen, und in denen das Stinging der α-Hydroxycarbonsäuren bzw. α-Ketocarbonsäuren verhindert oder drastisch vermindert ist.

Der pH-Wert der Erfindungsgemäßen Zubereitungen kann vorteilhaft im sauren Bereich eingestellt werden, wobei der pH-Bereich von 3,5 - 7 bevorzugt wird, besonders bevorzugt von 4 - 5.

Erfindungsgemäße Zubereitungen können auch vorteilhaft als Sonnenschutzmittel dienen.

Emulsionen gemäß der Erfindung z.B. in Form einer Sonnenschutzcreme oder einer Sonnenschutzmilch sind vorteilhaft und enthalten z.B. die genannten Fette, Öle, Wachse und anderen Fettkörper, sowie Wasser und einen Emulgator, wie er üblicherweise für einen solchen Typ der Formulierung verwendet wird.

Die UVB-Filter können öllöslich oder wasserlöslich sein. Vorteilhafte öllösliche UVB-Filter sind z.B.:
- 3-Benzylidencampher-Derivate, vorzugsweise 3-(4-Methylbenzyliden)campher, 3-Benzylidencampher;
- 4-Aminobenzoesäure-Derivate, vorzugsweise 4-(Dimethylamino)-benzoesäure(2-ethylhexyl)ester, 4-(Dimethylamino)benzoesäureamylester;
- Ester der Zimtsäure, vorzugsweise 4-Methoxyzimtsäure(2-ethylhexyl)ester, 4-Methoxyzimtsäureisopentylester;
- Ester der Salicylsäure, vorzugsweise Salicylsäure(2-ethylhexyl)ester, Salicylsäure(4-isopropylbenzyl)ester, Salicylsäurehomomenthylester;
- Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;
- Ester der Benzalmalonsäure, vorzugsweise 4-Methoxybenzalmalonsäuredi(2-ethylhexyl)ester;
- 2,4,6-Trianilino-(p-carbo-2'-ethyl-1'-hexyloxy)-1,3,5-triazin.

Vorteilhafte wasserlösliche UVB-Filter sind z.B.:
- Salze der 2-Phenylbenzimidazol-5-sulfonsäure wie ihr Natrium-, Kalium- oder ihr Triethanolammonium-Salz, sowie die Sulfonsäure selbst;
- Sulfonsäure-Derivate von Benzophenonen, vorzugsweise 2-Hydroxy-4-methoxybenzophenon-5-sulfonsäure und ihre Salze;
- Sulfonsäure-Derivate des 3-Benzylidencamphers, wie z.B. 4-(2-Oxo-3-bornylidenmethyl)benzolsulfonsäure, 2-Methyl-5-(2-oxo-3-bornylidenmethyl)sulfonsäure und ihre Salze.

Vorteilhaft können die erfindungsgemäßen Zubereitungen außerdem Substanzen enthalten, die UV-Strahlung im UVA-Bereich absorbieren. UVA-Filter, die erfindungsgemäß vorteilhaft verwendet werden können, sind beispielsweise Derivate des Dibenzoylmethans, insbesondere 1-(4'-tert.Butylphenyl)-3-(4'-methoxyphenyl)propan-1,3-dion und um 1-Phenyl-3-(4'-isopropylphenyl)propan-1,3-dion.

Die Gesamtmenge der UVA-Filtersubstanzen kann vorteilhaft 0,1 Gew.-% bis 30 Gew.-%, vorzugsweise 0,5 bis 10 Gew.-%, insbesondere 1 bis 6 Gew.-% betragen, bezogen auf das Gesamtgewicht der Zubereitung, um kosmetische Zubereitungen zur Verfügung zu stellen, die die Haut vor dem gesamten Bereich der ultravioletten Strahlung schützen, und in denen das Stinging der α-Hydroxycarbonsäuren bzw. α-Ketocarbonsäuren verhindert oder drastisch vermindert ist.

Vorteilhaft sind auch solche kosmetische und dermatologische Zubereitungen, die in der Form eines Sonnenschutzmittels, eines Pré-Soleil- oder Après-Soleil-Produktes vorliegen. Vorteilhaft enthalten diese zusätzlich mindestens einen UVA-Filter und/oder mindestens einen UVB-Filter.

Ferner sind auch solche kosmetische und dermatologische Zubereitungen besonders vorteilhaft, die in der Form eines Sonnenschutzmittels, eines Pré-Soleil- oder Après-Soleil-Produktes vorliegen und zusätzlich zu dem oder den UVA-Filtern und/oder dem oder den UVB-Filtern ein oder mehrere Antioxidantien enthalten.

Die Gesamtmenge der Antioxidantien kann vorteilhaft 0,1 Gew.-% bis 30 Gew.-%, vorzugsweise 0,5 bis 10 Gew.-%, insbesondere 1 bis 6 Gew.-% betragen, bezogen auf das Gesamtgewicht der Zubereitung.

Im den folgenden Beispielen werden vorteilhafte Verkörperungen der vorliegenden Erfindung aufgeführt.

### Beispiel 1

| Sonnencrème, O/W, Lichtschutzfaktor 20 | |
|---|---|
| | Gew.-% |
| Cyclomethicon | 3,00 |
| Glycerylstearat + PEG-30 Stearat | 2,00 |
| Wollwachsalkohol | 0,10 |
| Glycerylstearat | 3,00 |
| Isopropylpalmitat | 2,00 |
| Octyldodecanol | 1,00 |
| C₁₂₋₁₅Alkylbenzoat | 2,00 |
| Glycerin | 3,00 |
| Cetylalkohol | 3,00 |
| Myristylmyristat | 2,00 |
| Octylmethoxycinnamat | 4,50 |
| Butylmethoxydibenzoylmethan | 2,00 |
| Phenylbenzimidazolsulfonsäure | 3,00 |
| Tocopherylacetat | 0,50 |
| EDTA-Lösung (20 %-ig) | 0,50 |
| NaOH (45 %-ig) | q.s. |
| Ethylalkohol | 4,00 |
| Konservierungsmittel | q.s. |
| Parfüm | q.s. |
| Hydrophobes TiO₂ | 2,00 |
| Milchsäure | 3,00 |
| Wasser, VES | ad 100,00 |

### Beispiel 2

| Sonnencrème, O/W, Lichtschutzfaktor 8 | |
|---|---|
| | Gew.-% |
| Cyclomethicon | 3,00 |
| Glycerylstearat + PEG-30 Stearat | 2,00 |
| Glycerylstearat | 3,00 |
| Isopropylpalmitat | 2,00 |
| Octyldodecanol | 3,00 |
| Glycerin | 3,00 |
| Cetylalkohol | 3,00 |
| Phenylbenzimidazolsulfonsäure | 1,00 |
| Octylmethoxycinnamat | 5,00 |
| Butylmethoxydibenzoylmethan | 0,50 |
| Tocopherylacetat | 0,50 |
| EDTA-Lösung (20 %-ig) | 0,50 |
| NaOH | q.s. |
| Ethylalkohol | 1,50 |
| Konservierungsmittel | q.s. |
| Parfüm | q.s. |
| Hydrophobes TiO₂ | 1,00 |
| Milchsäure | 0,50 |
| Wasser, VES | ad 100,00 |

### Beispiel 3

| Sonnenmilch, O/W | |
|---|---|
| | Gew.-% |
| Trilaureth-4 Phosphat | 0,75 |
| Triceteareth-4 Phosphat | 1,00 |
| Glycerylstearat + PEG-100 Stearat | 1,00 |
| Glycerylstearat + Ceteareth-20 | 0,80 |
| Glyceryllanolat | 0,50 |
| Isopropylpalmitat | 3,00 |
| Glycerin-Caprylat/Caproat | |
| ("Caprylic/Capric Triglyceride") | 5,00 |
| Cetylalkohol | 1,00 |
| Xanthangummi | 0,30 |
| Octylmethoxycinnamat | 6,00 |
| Butylmethoxydibenzoylmethan | 0,50 |
| Methylbenzylidencampher | 3,00 |
| Phenylbenzimidazolsulfonsäure | 4,00 |
| Butylhydroxytoluol | 0,06 |
| EDTA-Lösung (20 %-ig) | 0,50 |
| NaOH | q.s. |
| Konservierungsmittel | q.s. |
| Milchsäure | 1,00 |
| Hydrophobes TiO₂ | 3,00 |
| Wasser, VES | ad 100,00 |

### Beispiel 4

| Sonnenmilch, O/W | |
|---|---|
| | Gew.-% |
| Cyclomethicon | 2,00 |
| Cetearylalkohol + PEG-40-hydriertes | |
| Rizinusöl + Natriumcetearylsulfat | 2,50 |
| Glyceryllanolat | 1,00 |
| Glycerin-Caprylat/Caproat | |
| ("Caprylic/Capric Triglyceride") | 2,00 |
| Octylstearat | 3,00 |
| Rizinusöl | 4,00 |
| Glycerin | 3,00 |
| Carbomer | 0,23 |
| Hydroxypropylmethylcellulose | 0,30 |
| PVP/Hexadecen Copolymer | 1,50 |
| Octylmethoxycinnamat | 4,00 |
| Butylmethoxydibenzoylmethan | 2,00 |
| Methylbenzylidencampher | 3,50 |
| Butylhydroxytoluol | 0,03 |
| EDTA-Lösung (20 %-ig) | 0,50 |
| NaOH | q.s. |
| Konservierungsmittel | q.s. |
| Parfüm | q.s. |
| Milchsäure | 2,00 |
| Hydrophobes TiO₂ | 1,50 |
| Wasser, VES | ad 100,00 |

### Beispiel 5

| Sonnenmilch, O/W | |
|---|---|
| | Gew.-% |
| Stearinsäure | 2,00 |
| Cetylpalmitat | 1,00 |
| Glycerin-Caprylat/Caproat | |
| ("Caprylic/Capric Triglyceride") | 2,00 |
| Sorbitanmonooleat | 1,00 |
| Sorbitanmonostearat | 1,50 |
| Paraffinöl, DAB 9 | 1,29 |
| Cetearylalkohol | 0,80 |
| Glycerin | 3,00 |
| 2-Hydroxy-4-methoxybenzophenon | 1,00 |
| Octylmethoxycinnamat | 5,00 |
| Methylbenzylidencampher | 1,00 |
| Butylhydroxytoluol | 0,06 |
| Simethicon | 0,20 |
| Polyethylenglycol-150 | 3,00 |
| Triethanolamin | 0,85 |
| Carbomer | 0,10 |
| Ethylalkohol | 3,00 |
| Konservierungsmittel | q.s. |
| Parfüm | q.s. |
| Milchsäure | 1,50 |
| Hydrophobes TiO₂ | 2,00 |
| Wasser, VES | ad 100,00 |

### Beispiel 6

| Sonnencrème, O/W, Lichtschutzfaktor 20 | |
|---|---|
| | Gew.-% |
| Cyclomethicon | 3,00 |
| Glycerylstearat + PEG-30 Stearat | 2,00 |
| Wollwachsalkohol | 0,10 |
| Glycerylstearat | 3,00 |
| Isopropylpalmitat | 2,00 |
| Octyldodecanol | 1,00 |
| C₁₂₋₁₅Alkylbenzoat | 2,00 |
| Glycerin | 3,00 |
| Cetylalkohol | 3,00 |
| Myristylmyristat | 2,00 |
| Octylmethoxycinnamat | 4,50 |
| Butylmethoxydibenzoylmethan | 2,00 |
| Phenylbenzimidazolsulfonsäure | 3,00 |
| Tocopherylacetat | 0,50 |
| EDTA-Lösung (20 %-ig) | 0,50 |
| NaOH (45 %-ig) | q.s. |
| Ethylalkohol | 4,00 |
| Konservierungsmittel | q.s. |
| Parfüm | q.s. |
| Hydrophobes TiO₂ | 2,00 |
| Brenztraubensäure | 3,00 |
| Wasser, VES | ad 100,00 |

### Beispiel 7

| Sonnencrème, O/W, Lichtschutzfaktor 8 | |
|---|---|
| | Gew.-% |
| Cyclomethicon | 3,00 |
| Glycerylstearat + PEG-30 Stearat | 2,00 |
| Glycerylstearat | 3,00 |
| Isopropylpalmitat | 2,00 |
| Octyldodecanol | 3,00 |
| Glycerin | 3,00 |
| Cetylalkohol | 3,00 |
| Phenylbenzimidazolsulfonsäure | 1,00 |
| Octylmethoxycinnamat | 5,00 |
| Butylmethoxydibenzoylmethan | 0,50 |
| Tocopherylacetat | 0,50 |
| EDTA-Lösung (20 %-ig) | 0,50 |
| NaOH | q.s. |
| Ethylalkohol | 1,50 |
| Konservierungsmittel | q.s. |
| Parfüm | q.s. |
| Hydrophobes TiO₂ | 1,00 |
| Brenztraubensäure | 0,50 |
| Wasser, VES | ad 100,00 |

### Beispiel 8

| Sonnenmilch, O/W | |
|---|---|
| | Gew.-% |
| Trilaureth-4 Phosphat | 0,75 |
| Triceteareth-4 Phosphat | 1,00 |
| Glycerylstearat + PEG-100 Stearat | 1,00 |
| Glycerylstearat + Ceteareth-20 | 0,80 |
| Glyceryllanolat | 0,50 |
| Isopropylpalmitat | 3,00 |
| Glycerin-Caprylat/Caproat | |
| ("Caprylic/Capric Triglyceride") | 5,00 |
| Cetylalkohol | 1,00 |
| Xanthangummi | 0,30 |
| Octylmethoxycinnamat | 6,00 |
| Butylmethoxydibenzoylmethan | 0,50 |
| Methylbenzylidencampher | 3,00 |
| Phenylbenzimidazolsulfonsäure | 4,00 |
| Butylhydroxytoluol | 0,06 |
| EDTA-Lösung (20 %-ig) | 0,50 |
| NaOH | q.s. |
| Konservierungsmittel | q.s. |
| Brenztraubensäure | 1,00 |
| Hydrophobes TiO₂ | 3,00 |
| Wasser, VES | ad 100,00 |

### Beispiel 9

| Sonnenmilch, O/W | |
|---|---|
| | Gew.-% |
| Cyclomethicon | 2,00 |
| Cetearylalkohol + PEG-40-hydriertes | |
| Rizinusöl + Natriumcetearylsulfat | 2,50 |
| Glyceryllanolat | 1,00 |
| Glycerin-Caprylat/Caproat | |
| ("Caprylic/Capric Triglyceride") | 2,00 |
| Octylstearat | 3,00 |
| Rizinusöl | 4,00 |
| Glycerin | 3,00 |
| Carbomer | 0,23 |
| Hydroxypropylmethylcellulose | 0,30 |
| PVP/Hexadecen Copolymer | 1,50 |
| Octylmethoxycinnamat | 4,00 |
| Butylmethoxydibenzoylmethan | 2,00 |
| Methylbenzylidencampher | 3,50 |
| Butylhydroxytoluol | 0,03 |
| EDTA-Lösung (20 %-ig) | 0,50 |
| NaOH | q.s. |
| Konservierungsmittel | q.s. |
| Parfüm | q.s. |
| Brenztraubensäure | 2,00 |
| Hydrophobes TiO₂ | 1,50 |
| Wasser, VES | ad 100,00 |

### Beispiel 10

| Sonnenmilch, O/W | |
|---|---|
| | Gew.-% |
| Stearinsäure | 2,00 |
| Cetylpalmitat | 1,00 |
| Glycerin-Caprylat/Caproat | |
| ("Caprylic/Capric Triglyceride") | 2,00 |
| Sorbitanmonooleat | 1,00 |
| Sorbitanmonostearat | 1,50 |
| Paraffinöl, DAB 9 | 1,29 |
| Cetearylalkohol | 0,80 |
| Glycerin | 3,00 |
| 2-Hydroxy-4-methoxybenzophenon | 1,00 |
| Octylmethoxycinnamat | 5,00 |
| Methylbenzylidencampher | 1,00 |
| Butylhydroxytoluol | 0,06 |
| Simethicon | 0,20 |
| Polyethylenglycol-150 | 3,00 |
| Triethanolamin | 0,85 |
| Carbomer | 0,10 |
| Ethylalkohol | 3,00 |
| Konservierungsmittel | q.s. |
| Parfüm | q.s. |
| Brenztraubensäure | 1,50 |
| Hydrophobes TiO₂ | 2,00 |
| Wasser, VES | ad 100,00 |

### Vergleichsbeispiel 1

| Sonnenmilch, O/W | |
|---|---|
| | Gew.-% |
| Cyclomethicon | 2,00 |
| Cetearylalkohol + PEG-40-hydriertes Rizinusöl + Natriumcetearylsulfat | 2,50 |
| Glyceryllanolat | 1,00 |
| Glycerin-Caprylat/Caproat ("Caprylic/Capric Triglyceride") | 2,00 |
| Octylstearat | 3,00 |
| Rizinusöl | 4,00 |
| Glycerin | 3,00 |
| Carbomer | 0,23 |
| Hydroxypropylmethylcellulose | 0,30 |
| PVP/Hexadecen Copolymer | 1,50 |
| Octylmethoxycinnamat | 4,00 |
| Butylmethoxydibenzoylmethan | 2,00 |
| Methylbenzylidencampher | 3,50 |
| Butylhydroxytoluol | 0,03 |
| Na₃HEDTA | 1,00 |
| NaOH | q.s. |
| Konservierungsmittel | q.s. |
| Parfüm | q.s. |
| Milchsäure | 2,00 |
| Wasser, VES | ad 100,00 |

### Versuchsbericht

Mit dem folgenden Versuchsbericht wird die Wirkung der erfindungsgemäßen Wirkstoffe demonstriert. Dazu wird die Zubereitung des Beispiels 4, gegenüber dem Vergleichsbeispiel 1 (mit Milchsäure, aber ohne hydrophobes TiO₂) in einem "Stingingtest" als "Stinger" analog Frosch und Kligman verwendet (Peter J. Frosch und Albert M. Kligman, a.a.O).

Es wurden acht freiwillige Probanden ausgewählt, deren hohe Empfindlichkeit gegenüber Milchsäure bekannt war. Alle Personen zeigten die erwartete Reaktion bei dem Vergleichsbeispiel. Fünf Personen teilten mit, daß die Zubereitung gemäß Beispiel 4 geringere oder gar keine "Stinging-Effekte" hervorrief als bei der O/W-Emulsion gemäß Vergleichsbeispiel 1. Bei drei Personen waren keine Unterschiede zwischen der Anwendung von Zubereitungen gemäß Beispiel 4 und Vergleichsbeispiel 1 festzustellen.

## Patentansprüche

1. Kosmetische oder dermatologische Zubereitungen mit einem Gehalt an einer wirksamen Menge an einer oder mehreren α-Hydroxycarbonsäuren der allgemeinen Formel 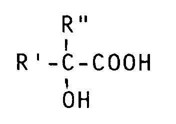 und/oder einer wirksamen Menge an einer oder mehreren α-Ketocarbonsäuren der allgemeinen Formel 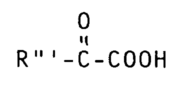 wobei jeweils R', R'' und R''' unabhängig voneinander gewählt werden aus der Gruppe
(b1) H- ,
(b2) verzweigtes oder unverzweigtes C₁₋₂₅-Alkyl-,
(b3) mit einer oder mehreren Carboxylgruppen und/oder Hydroxygruppen und/oder Aldehydgruppen und/oder Oxogruppen (Ketogruppen) substituiertes verzweigtes oder unverzweigtes C₁₋₂₅-Alkyl-
(b4) Phenyl-,
(b5) mit einer oder mehreren Carboxylgruppen und/oder Hydroxygruppen und/oder verzweigten und/oder unverzweigten C₁₋₂₅-Alkylgruppen substituiertes Phenyl-,
oder wobei das α-Kohlenstoffatom der α-Hydroxycarbonsäure mit R' und R'' zusammen eine
(b6) unsubstituierte Cycloalkylgruppe mit 3 bis 7 Ringatomen oder eine
(b7) mit einer oder mehreren Carboxylgruppen und/oder Hydroxygruppen und/oder Oxogruppen (Ketogruppen) und/oder verzweigten und/oder unverzweigten C₁₋₂₅-Alkylgruppen substituierte Cycloalkylgruppe mit 3 bis 7 Ringatomen
ausbildet und
wobei die α-Hydroxycarbonsäure oder die α-Hydroxycarbonsäuren oder die α-Ketocarbonsäure oder die α-Ketocarbonsäuren gegebenenfalls in Form ihrer physiologisch verträglichen Salze und/oder Ethylester und/oder Methylester vorliegen können
und hydrophoben anorganischen Mikropigmenten.

2. Zubereitungen nach Anspruch 1, dadurch gekennzeichnet, daß die α-Hydroxycarbonsäuren gewählt werden aus folgenden Substanzklassen:
(b2) α-Hydroxyfettsäuren, wobei diese wiederum besonders vorteilhaft aus der Gruppe der C₁₀₋₁₈-Alkylcarbonsäuren gewählt werden,
(b3) α-Hydroxyzuckersäuren, aliphatische α-Hydroxyfruchtsäuren,
(b4) unsubstituierte aromatische α-Hydroxycarbonsäuren (z.B. Mandelsäure) bzw.
(b5) substituierte aromatische α-Hydroxycarbonsäuren.

3. Zubereitungen nach Anspruch 2, dadurch gekennzeichnet, daß die α-Hydroxyfettsäuren gewählt werden gewählt aus der Gruppe
- α-Hydroxycarbonsäuren, gemäß der Formel 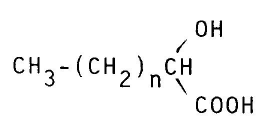 und/oder
- α-Hydroxy-isocarbonsäuren, gemäß der Formel 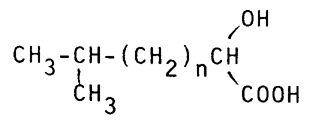 und/oder
- α-Hydroxy-anteisocarbonsäuren, gemäß der Formel 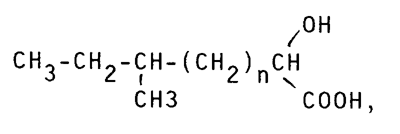 wobei n jeweils eine Zahl von 7 bis 31 darstellt,

4. Zubereitungen nach Anspruch 2, dadurch gekennzeichnet, daß die aliphatischen α-Hydroxyfruchtsäuren gewählt werden aus der Gruppe Äpfelsäure, Milchsäure, Citronensäure, Weinsäure.

5. Zubereitungen nach Anspruch 1, dadurch gekennzeichnet, daß als α-Ketocarbonsäure die Brenztraubensäure gewählt wird.

6. Zubereitungen nach Anspruch 1, dadurch gekennzeichnet, daß das hydrophobierte anorganische Pigment auf TiO₂ basiert.

7. Zubereitungen nach Anspruch 1, dadurch gekennzeichnet, daß sie einen Gehalt von 0,1 bis 10 Gew.-%, insbesondere 0,5 - 5,0 Gew.-%, an α-Hydroxycarbonsäuren bzw. α-Ketocarbonsäuren und einen Gehalt von 0,1 bis 10 Gew.-%, insbesondere 0,5 - 5,0 Gew.-% an hydrophoben anorganischen Pigmenten aufweisen, jeweils bezogen auf das Gesamtgewicht der Zusammensetzung.

8. Verwendung von hydrophoben anorganischen Pigmenten zur Verhinderung des Stingings von α-Hydroxycarbonsäuren und/oder α-Ketocarbonsäuren in kosmetischen oder dermatologischen Zubereitungen.
